Europäisches Patentamt

European Patent Office

Office européen des brevets

(19)

(11) Publication number: **0 040 762**
**B1**

(12) **EUROPEAN PATENT SPECIFICATION**

(45) Date of publication of patent specification: **18.04.84**

(51) Int. Cl.³: **C 07 C 87/62, C 07 C 85/04**

(21) Application number: **81103693.8**

(22) Date of filing: **13.05.81**

(54) **Process for the preparation of N-di-n-propyl-2,6-dinitro-4-trifluoromethylaniline with low content of nitrosamines.**

(30) Priority: **15.05.80 IT 2207380**

(43) Date of publication of application:
**02.12.81 Bulletin 81/48**

(45) Publication of the grant of the patent:
**18.04.84 Bulletin 84/16**

(84) Designated Contracting States:
**BE CH DE FR GB LI NL**

(56) References cited:
**EP - A - 0 019 281**
**FR - A - 2 051 301**
**FR - A - 2 165 551**
**US - A - 4 120 905**

(73) Proprietor: **Montedison S.p.A.**
**Patents & Licensing Dept. Foro Buonaparte, 31**
**P.O. Box 10528**
**I-20121 Milan (IT)**

(72) Inventor: **Bornengo, Mario, Dipl.-Chem.**
**206, Via Massa-Avenza**
**Massa (IT)**
Inventor: **Bacciarelli, Sergio, Dr.**
**Via Casano di Orzonovo**
**I-19034 Orzonovo (La Spezia) (IT)**

(74) Representative: **Schmied-Kowarzik, Volker, Dr. et al,**
**Siegfriedstrasse 8**
**D-8000 München 40 (DE)**

The file contains technical information submitted after the application was filed and not included in this specification

Courier Press, Leamington Spa, England.

# 0 040 762

Process for the preparation of n-di-n-propyl-2,6-dinitro-4-trifluoromethylaniline with
low content of nitrosamines

N-di-n-propyl-2,6-dinitro-4-trifluoromethyl-aniline is a herbicide marketed by Eli Lilly Co., an American company, under the commercial name of Trifluralin, its use being protected by US—PS 3 257 190.

According to said patent, Trifluralin may be prepared from 4-trifluoromethyl-2,6-dinitrochlorobenzene, by heating it with an excess of di-n-propylamine sufficient for blocking the hydrochloric acid that develops thereby; there is formed di-n-propylamine hydrochloride which is then removed by filtering and from which may be recovered the n-propylamine to be re-cycled back to the reaction with 4-trifluoromethyl-2,6-dinitrochlorobenzene.

Under such conditions, as by-products, there are obtained considerable quantities of nitrosamines (which may attain up to 250 parts per million), which are considered dangerous for warm blooded animals, and particularly for man, so much so that some laws put limits to their concentration in herbicides to be used on rural soils.

According to CA—PS 1 057 774 by Eli Lilly, the nitrsoamines present in the end product may be considerably lowered if the Trifluralin is put into contact in a liquid phase and at temperatures preferably below 120°C, with bromine, chlorine, N-bromosuccinimide, N-chlorosuccinimide, bromine chloride, pyridine perbromide and bromopyridine per bromide; according to CA—PS 1,062,729 one obtains the same result by treating it, under the same conditions of the preceding patent, with $PCl_3$, $PCl_5$, $PBr_3$ and $POCl_3$.

According to DE—OS 2,816,637 (by American Cyanamid) the nitrosamines are removed from the Trifluralin by reaction with an aldehyde or a ketone in the presence of HCl or HBr; according to BE—PS 869,042 by Eli Lilly, the Trifluralin is treated with a 20% HCl and ethanol at 90°C.

Thus, object of this invention is that of providing a process for obtaining a Trifluralin that will yield a product practically free of nitrosamines.

Another object of this invention is that of providing a simpler process for the synthesis of Trifluralin, in which the reaction by products be more limited.

These and still other objects may be achieved if the reaction process between 4-trifluoromethyl-2,6-dinitro-chlorobenzene and di-n-propylamine is conducted by contemporaneously but separately introducing into the aqueous 4-trifluoromethyl-2,6-dinitro-chlorobenzene the di-n-propylamine and an aqueous alkali metal hydroxide or an alkali carbonate solution so that the pH shall remain always comprised between 7 and 7.5; in that the temperature is regulated in such way so as not to exceed 70°C; and operating in the presence of at least 50% by weight of water in the reaction mass.

Under such conditions, it is supposed that the di-n-propylamine hydrochloride will not be formed (in fact, in order that hydrochloride be formed, it would be necessary to lower the pH value to 4.3 — 4.5); the low temperature will hinder a loss of di-n.propylamine, and all these conditions will lead to a point where the content in nitrosamine in the end product will at most reach 1 ppm.

The reaction forming the alkali chloride is an exothermic one and must therefore be kept under control by means of a suitable cooling system. The presence of water is, moreover, necessary for maintaining the sodium chloride present in solution and for its removal.

The quantities of nitrosamine found in the herbicide prepared according to the above illustrated method, vary from 0.1 ppm to 1 ppm. In order to achieve such results it is necessary to keep strictly to the indicated operational conditions; a drop in the pH value into the acid field may cause an increase of the nitrosamine to more than 1 ppm; an increase of the temperature above 100°C in an alkaline medium leads to losses of n-propylamine; an increase of the pH above 7.5 gives unsatisfactory yields because of the increase of secondary reactions.

In order to still better illustrate this invention, there will be given in the following a series of examples.

Example 1:
Into a 4 l flask were loaded 1200 g of 4-trifluoromethyl-2,6-dinitro-chlorobenzene, 2 l of water, whereupon the temperature was brought up to 55°C under stirring.

Through two drawing pipes were then contemporaneously additioned 450 g of 99% di-n-propylamine and 355 g of 40% by weight NaOH; by means of a drawing electrode the flow was adjusted in such a way that the pH remained constant between 7 and 7.5.

During the addition, the flask was cooled by water so that the inside temperature did not excced 55°C. On completion of the addition, the reaction mass was heated up to 70°C for about 3 hours, while still maintaining the pH at 7.2 (possibly adding a few drops of 40% by weight NaOH).

The stirring and heating were then stopped and the organic phase (the lower one) is allowed to mix through, after which the floating mother liquors were syphoned away. The mixture was then washed twice with 1 l of warm water (55%), each time under stirring, until attaining neutrality.

The molten product gradually coagulated. The yield in N-di-n.propyl-2,6-dinitro-4-trifluoromethylaniline attained 97% by weight calculated on the starting 4-trifluoromethyl-2,6-dinitrochlorobenzene. Purity, amounted to 95% while the nitrosamines present amounted to 0.1 ppm.

2

### Example 2 (comparison):

Under the same operational conditions as those of example 1, the reaction between di-n-propylamine and 4-trifluoromethyl-2,6-chlorobenzene was carried out by adding sodium hydroxide solution after having fed in all the di-n-propylamine.

The end yield amounted to 95% by weight, but the nitrosamines present were just 250 ppm.

### Example 3 (comparison):

Under the same conditions as those of example 1, conducting the reaction by the contemporaneous addition of di-n-propylamine and sodium hydroxide solution, but allowing the temperature to rise spontaneously during the addition, the purity of the product thus obtained dropped to 92% while the nitrosamines amounted to 10 ppm.

### Example 4 (comparison):

Under the same operational conditions as those of example n° 1, into the reactor were loaded 1200 g of d4-trifluoromethyl-2,6-dinitro-chlorobenzene which were then heated up to 55°C in the presence of 40% of water. Thereupon was added the di-n-propylamine and contemporaneously the sodim hydroxide solution, maintaining the pH still at a value between 7.2 and 7.5.

The content in nitrosamine in the end product rose to 10 ppm, while the yield amounted to 95% by weight and product showed a purity of 94%.

**Claim**

Process for obtaining herbicide N-di-n-propyl-2,6-dinitro-4-trifluoromethylaniline with a content in nitrosamine lower than 1 ppm by reacting 4-trifluoromethyl-2,6-dinitro-chlorobenzene and di-n-propylamine, characterized in that the reaction is conducted by contemporaneously but separately introducing into the aqueous 4-trifluoromethyl-2,6-dinitrochlorobenzene the di-n-propylamine and an aqueous alkali metal hydroxide or an alkali carbonate solution so that the pH shall remain always comprised between 7 and 7.5; in that the temperature is regulated in such a way so as not to exceed 70°C; and operating in the presence of at least 50% by weight of water in the reaction mass.

**Revendication**

Procédé d'obtention d'un herbicide à base de N-di-n-propyl-2,6-dinitro-4-trifluorométhylaniline ayant une teneur en nitrosamine inférieure à 1 ppm par réaction de 4-trifluorométhyl-2,6-dinitro-chlorobenzène et de di-n-propylamine, caractérisé en ce que la réaction est conduite en introduisant simultanément, mais séparément, dans le 4-trifluorométhyl-2,6-dinitrochlorobenzène aqueux la di-n-propylamine et une solution aqueuse d'hydroxde de métal alcalin ou d'un carbonate alcalin, de telle sorte que le pH reste toujours compris entre 7 et 7,5, en ce que la température est réglée de façon à ne pas dépasser 70°C et en ce que l'on opère en présence d'au moins 50% en poids d'eau dans la masse réactionnelle.

**Patentanspruch**

Verfahren zur Herstellung von herbizidem N-di-n-propyl-2,6-dinitro-4-trifluormethylanilin mit einem Gehalt an Nitrosanim unter 1 ppm durch Umsetzung von 4-Trifluormethyl-2,6-dinitro-chlorbenzol und Di-n-propylamin, dadurch gekennzeichnet, daß die Reaktion durch gleichzeitige, jedoch getrennte Einführung des Di-n-propylamins und einer wässrigen Alkalimetallhydroxid- oder einer Alkalicarbonatlösung in das wässrige 4-Trifluormethyl-2,6-dinitro-chlorbenzol erfolgt, so daß der pH Wert immer zwischen 7 und 7,5 bleibt, daß die Temperatur in solcher Weise geregelt wird, daß sie 70°C nicht übersteigt und daß man in Anwesenheit von mindestens 50 Gew.-% Wasser in der Reaktionsmasse arbeitet.